## Europäisches Patentamt
## European Patent Office
## Office européen des brevets

(19)

(11) Veröffentlichungsnummer: **0 048 946**
**B1**

(12) **EUROPÄISCHE PATENTSCHRIFT**

(45) Veröffentlichungstag der Patentschrift:
**28.09.83**

(21) Anmeldenummer: **81107549.8**

(22) Anmeldetag: **23.09.81**

(51) Int. Cl.³: **C 07 C  67/055,** C 07 C  69/15,
C 07 C  69/155

(54) **Verfahren zur Herstellung von ungesättigten Carbonsäureestern.**

(30) Priorität: **26.09.80  DE 3036421**

(43) Veröffentlichungstag der Anmeldung:
**07.04.82 Patentblatt 82/14**

(45) Bekanntmachung des Hinweises auf die Patenterteilung:
**28.09.83 Patentblatt 83/39**

(84) Benannte Vertragsstaaten:
**BE DE FR GB IT NL**

(56) Entgegenhaltungen:
**US-A-3 607 916**
**US-A-3 622 620**

(73) Patentinhaber: **WACKER-CHEMIE GMBH,
Prinzregentenstrasse 22, D-8000 München 22 (DE)**
Patentinhaber: **HOECHST AKTIENGESELLSCHAFT,
Postfach 80 03 20, D-6230 Frankfurt am Main 80 (DE)**

(72) Erfinder: **Dempf, Dominik, Dr. Dipl.-Chem.,
Schifferlehnerstrasse 1, D-8261 Mehring (DE)**
Erfinder: **Schmidhammer, Ludwig, Dr. Dipl.-Chem.,
Pappelweg 5, D-8261 Halming (DE)**
Erfinder: **Dummer, Gerhard, Dipl.-Ing., Lohnerstrasse 12,
D-8261 Burgkirchen (DE)**
Erfinder: **Roscher, Günter, Dr. Dipl.-Chem.,
Altkönigstrasse 7, D-6233 Kelkheim (DE)**
Erfinder: **Schmidt, Karl-Heinz, Dr. Dipl.-Chem.,
Taubenberg 86, D-6270 Idstein (DE)**
Erfinder: **Selbertinger, Ernst, Ing.-grad.,
Bahnhofstrasse 1, D-8261 Markt 1 (DE)**
Erfinder: **Strasser, Rudolf, Dr. Dipl.-Chem.,
Lindacherstrasse 58, D-8263 Burghausen (DE)**

## Verfahren zur Herstellung von ungesättigten Carbonsäureestern

Die Erfindung betrifft ein Verfahren zur Herstellung von ungesättigten Carbonsäureestern durch Umsetzung von Olefinen oder Cyclo-Olefinen mit Sauerstoff und Carbonsäure in der Gasphase in Gegenwart von Elemente der 8. Nebengruppe des Periodensystems und/oder deren Verbindungen, sowie Alkaliacetat enthaltendem Katalysator bei Temperaturen von 100 bis 250° C und Drücken von 0,5 bis 21 bar absolut.

Die Herstellung ungesättigter Carbonsäureester durch Umsetzung von Olefin und Sauerstoff mit Carbonsäure an Edelmetallkontakten ist an sich bekannt (vgl. hierzu DE-PS 1 443 882). Es wurden auch bereits eine Reihe von Verbesserungsvorschlägen veröffentlicht: so werden beispielsweise gemäß DE-AS 1 244 760 und DE-OS 2 315 037 Maßnahmen zur Erhöhung der Ester-Raum-Zeit-Leistung des Verfahrens beschrieben.

Neben der Katalysatoreffizienz bestimmen jedoch weitere Faktoren die Wirtschaftlichkeit des Verfahrens, wie die Lebensdauer des Edelmetallkatalysators und die Olefinausbeute des Prozesses. So beträgt beispielsweise für den Fall der Herstellung von Vinylacetat gemäß DE-AS 1 277 249 die Verbrennungsrate von Ethylen zu wertlosem $CO_2$ 7 bis 13%, bezogen auf umgesetztes Ethylen.

Ferner lehrt die US-PS 3 607 916 ein Flüssigphasen-Verfahren zur Herstellung von Vinylacetat wonach dem Reaktionsgemisch Chlor-Essigsäuren zugesetzt werden. Die sinnvollen Mengen an Chlor-Essigsäuren betragen 100−1000 Mol-%, bezogen auf die Menge aktiven Katalysatormaterials. Der Einsatz derart hoher Mengen an Fremdsubstanz verbietet sich jedoch bei Gasphasen/Festkörper-Reaktionen sowohl aus wirtschaftlichen als auch aus verfahrenstechnischen Gründen. Sie würden darüber hinaus zu einer Desaktivierung des Trägerkatalysators führen.

Aufgabe der Erfindung war es, die Verbrennungsrate eingesetzten Olefins zu senken und weiter die Lebensdauer des Katalysatorsystems zu verlängern.

Es wurde nun überraschenderweise gefunden, daß der Zusatz organischer Chlorverbindungen zum Reaktionssystem diese Aufgabe löst.

Gegenstand der Erfindung ist ein Verfahren zur Herstellung ungesättigter Carbonsäureester, das dadurch gekennzeichnet ist, daß die Umsetzung in Gegenwart von 1 bis 8000 Gew.-ppm, bezogen auf eingesetzte Carbonsäure, an solchen organischen Chlorverbindungen, einzeln oder im Gemisch, vorgenommen wird, die ausgewählt sind aus der Gruppe

a)   der α-Chlorcarbonsäuren mit 1 bis 4 Kohlenstoffatomen und deren Estern von Alkoholen mit 1 bis 4 Kohlenstoffatomen
b)   der α-Chloraldehyde mit 1 bis 4 Kohlenstoffatomen,
c)   der Chlorderivate des Methans mit 1 bis 4 Chloratomen,
d)   der Chlorderivate des Methans mit 1 bis 3 Chloratomen, die zumindest ein Fluoratom enthalten, und
e)   der Chlorderivate des Ethans und des Propans, die zumindest 2 Chloratome enthalten.

Die erfindungsgemäß einzusetzenden Chlorverbindungen werden bevorzugt in Mengen von 10 bis 100 Gew.-ppm, bezogen auf Carbonsäuremenge, eingesetzt.

Es ist weiterhin bevorzugt, daß die Zugabe der erfindungsgemäßen organischen Chlorverbindungen kontinuierlich erfolgt.

Die Umsetzung von Olefin oder Cyclo-Olefin mit Sauerstoff und Carbonsäure erfolgt nach dem erfindungsgemäßen Verfahren in an sich bekannter Weise bei Temperaturen von 100 bis 250° C in der Gasphase und Drücken von 0,5 bis 21 bar abs. in Gegenwart von Elemente der achten Nebengruppe des Periodensystems und/oder deren Verbindungen, sowie Alkaliacetat enthaltendem Katalysator.

Das zur Reaktion eingesetzte Gemisch enthält zumeist einen mehrfachen Überschuß an Olefin. Der Umsatz zum entsprechenden ungesättigten Carbonsäureester ist aus diesem Grunde, bezogen auf eingesetztes Olefin, nicht quantitativ. Nicht umgesetztes Olefin wird deshalb zusammen mit Beimengungen von u. a. Kohlendioxid, Stickstoff, Argon und Restsauerstoff im Kreislauf geführt. Vor Reaktor wird nach Maßgabe des Verbrauchs Olefin in das System eingeschleust. Das Gemisch durchströmt anschließend einen, beispielsweise als Kolonne ausgebildeten Carbonsäuresättiger, wobei durch entsprechende Temperaturführung die Soll-Carbonssäuremenge eingestellt wird. Die Carbonsäure ist mit 1 bis 8000 ppm an erfindungsgemäßen organischen Chlorverbindungen versetzt. Falls ewünscht, kann die Zudosierung der erfindungsgemäßen organischen Chlorverbindungen jedoch auch an anderer Stelle, jedoch vor Reaktor, erfolgen. Über eine Mischdüse wird weiterhin verbrauchter Sauerstoff zugeführt — im allgemeinen in Mengen bis zu 7 Vol.-%, bezogen auf das Gesamtgemisch. Das Gemisch gelangt schließlich, günstigerweise noch mit Aktivatorlösung beauschlagt, in einen Reaktor, der mit Elemente der achten Nebengruppe des Periodensystems und/oder deren Verbindungen, sowie Alkaliacetat enthaltendem Katalysator beschickt ist. Das dem Reaktor verlassende Gemisch enthält als wesentliche Bestandteile ungesättigten Carbonsäureester, nicht umgesetztes Olefin, Carbonsäure, Restsauerstoff, Kohlendioxid, Wasser sowie Inerte. Die Trennung des Gemisches erfolgt zunächst über einen Kondensationsschritt, in dem Carbonsäureester, Carbonsäure und Wasser abgetrennt werden. Das verbleibende Gasgemisch wird noch einer Pottaschewäsche zur Entfernung von $CO_2$ unterzogen. Ferner werden zur Aufrechterhaltung stationärer Bedingungen Inerte, wie Stickstoff, Argon u. a., über einen Teilstrom ausgeschleust. Schließlich wird das verbleibende Gasgemisch in den Kreislauf zurückgeführt.

Die vorstehende Beschreibung des Verfahrens erfolgte lediglich beispielhaft anhand der Verhältnisse, wie sie zur Durchführung des Verfahrens im Rahmen einer großtechnischen Produktion vorgenommen wird.

Der Erfindung liegt die Erkenntnis zugrunde, daß die Umsetzung von Olefinen oder Cyclo-Olefinen mit Sauerstoff und Carbonsäure in der Gasphase unter den anspruchsgemäßen Reaktionsbedingungen die Verbrennungsrate von Olefin erniedrigt und die Lebensdauer der Elemente der achten Nebengruppe des Periodensystems und/oder deren Verbindungen, sowie Alkaliacetat enthaltenden Katalysators erhöht. Diese Vorteile lassen sich ebenso mit sich in diesem Rahmen haltenden Modifikationen des Verfahrens erzielen.

Beispiele für erfindungsgemäß einzusetzende organische Chlorverbindungen sind:

Chloressigsäure, Dichloressigsäure, Trichloressigsäure, $\alpha$-Chlorpropionsäure, $\alpha$-Chlorbuttersäure, $\alpha$-Chlor-isobuttersäure, Chloressigsäuremethylester, Chloressigsäureethylester, Dichloressigsäureethylester, Chloressigsäurepropylester, Chloressigsäurebutylester, $\alpha$-Chlorpropionsäuremethylester, $\alpha$-Chlorpropionsäureethylester, Chloracetaldehyd, Dichloracetaldehyd, Trichloracetaldehyd, $\alpha$-Chlorpropionaldehyd, $\alpha$-Chlorbutyraldehyd, $\alpha$-Chlor-isobutyraldehyd, Methylchlorid, Methylenchlorid, Chloroform, Tetrachlorkohlenstoff, Dichlordifluormethan, Trichlorfluormethan, Chlor-trifluormethan, 1.1-Dichlorethan, 1.2-Dichlorethan, 1.1.1-Trichlorethan, 1.1.2-Trichlorethan, 1.1.1.2-Tetrachlorethan, 1.1.2.2-Tetrachlorethan, Pentachlorethan, Hexachlorethan, 1.2-Dichlorpropan, 1.3-Dichlorpropan, 1.1.2-Trichlorpropan und andere.

Als Olefine oder Cyclo-Olefine lassen sich erfindungsgemäß solche einsetzen, die unter den verfahrensgemäßen Bedingungen gasförmig sind. Beispiele sind Ethylen, Propylen, Butylen, Isobutylen, Penten, Hexen, Cyclo-Penten, Cyclo-Hexen und andere, insbesondere Ethylen.

Beispiele für Carbonsäuren sind Essigsäure, Propionsäure und andere.

Erfindungsgemäß werden bevorzugt Trägerkatalysatoren eingesetzt. Die Katalysatoren enthalten Elemente der achten Nebengruppe des Periodensystems und/oder deren Verbindungen. Beispiele sind die Elemente Palladium, Platin, Gold und andere, sowie deren Carbonsäuresalze, insbesondere deren Acetate. Ferner enthalten die Katalysatoren Alkaliacetat. Beispiele sind die Acetate des Lithiums, des Natriums, des Kaliums, des Rubidiums und des Cäsiums. Weiterhin können die Katalysatoren Zusätze zweiwertiger wie Mangan oder Cadmium enthalten.

Als Aktivatorlösung haben sich insbesondere wäßrige Kaliumacetatlösungen bewährt.

Beispiele für erfindungsgemäß herzustellende ungesättigte Carbonsäureester sind Vinylacetat, Allylacetat, Butenylacetat, Pentenylacetat, Hexenylacetat, Cyclopentenylacetat, Cyclohexenylacetat, Vinylpropionat, Vinylbutyrat, Vinylisobutyrat, Allylpropionat, Allylbutyrat u. a., insbesondere Vinylacetat.

Die zur Einschleusung der erfindungsgemäß umzusetzenden organischen Chlorverbindungen in das Reaktionssystem zu treffenden Maßnahmen sind dem Fachmann geläufig. Beispiele für solche Maßnahmen sind Eindüsen, Einspritzen und dergleichen. Ebenso können Extraktionstechniken angewendet werden.

Es bietet sich an, die Chlorverbindungen zusammen mit anderen Reaktionskomponenten einzuschleusen, beispielsweise zusammen mit der umzusetzenden Carbonsäure oder zusammen mit der Aktivatorlösung.

Die Zugabe kann kontinuierlich oder diskontinuierlich erfolgen.

Bei kontinuierlicher Zugabe der erfindungsgemäß einzusetzenden organischen Chlorverbindungen genügen Mengen, die im unteren anspruchsgemäßen Bereich liegen, vorzugsweise 10—100 ppm, bezogen auf eingesetzte Carbonsäuremenge. Zweckmäßigerweise wird die Carbonsäure in der gewünschten Menge mit organischer Chlorverbindung versetzt.

Bei diskontinuierlicher Zugabe werden zumeist die höheren anspruchsgemäßen Mengen eingesetzt. Die Notwendigkeit der Nachchargierung kann beispielsweise am ansteigenden $CO_2$-Gehalt der Reaktionsprodukte abgelesen werden.

Nach dem erfindungsgemäßen Verfahren gelingt es, die Verbrennungsrate von Olefin zu reduzieren. Demnach steigt sowohl die Olefin- als auch die Sauerstoffausbeute. Weiterhin wird die Lebensdauer des Katalysators bei erhöhter Katalysatoreffizienz verlängert.

Die Erfindung wird nun anhand von Beispielen und Vergleichsbeispielen näher erläutert:

0 048 946

Beispiel 1

Herstellung von Vinylacetat

Es wird ein Trägerkatalysator auf Basis von Bentonit eingesetzt mit aktiven Bestandteilen, jeweils in Acetatform, von 2,2 Gew.-% Palladium, 1,7 bis 1,9 Gew.-% Cadmium, 0,07 Gew.-% Mangan und 1,9 Gew.-% Kalium, jeweils bezogen auf Katalysatormenge. Es werden 19 m³ des oben beschriebenen Kontaktes in den Reaktor eingefüllt. Vor Reaktor werden noch kontinuierlich etwa 15 kg/h einer 2-gew.-%igen wäßrigen Lösung von Kaliumacetat eingedüst. Das über den Kontakt geleitete Gasgemisch hat folgende Zusammensetzung:

63 Vol.-%  Ethylen
12 Vol.-%  Essigsäure + erfindungsgemäße organische Chlorverbindungen
8 Vol.-%  $CO_2$
6 Vol.-%  Sauerstoff
11 Vol.-%  Inerte (Stickstoff, Argon, Ethan) und Wasser.

Die oben beschriebene Gasmenge beträgt 56 000 Nm³/h.
Die Essigsäure wird durch Sättigen des Kreisgases bei einer Temperatur von 115°C in das System eingebracht.
Der zum Essigsäuresättiger zufließenden Essigsäure werden 30 Gew.-ppm Monochloressigsäure zugesetzt. Die Temperatur im Katalysatorbett wird auf 165°C gehalten mit Hilfe einer mit Wasserdampf betriebenen Reaktoraußenmantelbeheizung. Der Systemdruck beträgt 8 bar und wird durch kontinuierliche Zugabe von 2780 Nm³/h Ethylen und 1847 Nm³/h Sauerstoff zum Kreisgas aufrechterhalten. Nach Verlassen des Reaktors wird das Gemisch einem Kondensationsschritt unterworfen, wobei 23,3 t/h Roh-Vinylacetat folgender Zusammensetzung gewonnen werden:

42,9 Gew.-%  Vinylacetat
47,2 Gew.-%  Essigsäure
9,9 Gew.-%  Wasser.

Es werden folgende spezifische Verbrauchswerte bzw. Ausbeuten erzielt:

Ethylen:  350,3 kg/t Vinylacetat, entsprechend 92,94 Gew.-% Ausbeute
Sauerstoff:  263,9 kg/t Vinylacetat, entsprechend 70,5 Gew.-% Ausbeute
Gebildetes $CO_2$: 49,1 kg/t Vinylacetat.

Der Ethylenumsatz beträgt 7,9 Gew.-%, bezogen auf eingesetztes Ethylen.
Der Ethylenverbrennungsanteil beträgt 0,36 Gew.-%, bezogen auf eingesetztes Ethylen und 4,5 Gew.-%, bezogen auf umgesetztes Ethylen.
Nach einer Betriebszeit von 17 000 Stunden ist die Katalysatoreffizienz, wie sie sich aus den oben gegebenen spezifischen Verbrauchswerten bzw. Ausbeuten ergibt, noch immer gegeben.

Vergleichsbeispiel 1

Es wird im wesentlichen unter den gleichen Temperatur- und Druckbedingungen und am gleichen Kontakt, wie in Beispiel 1 beschrieben, Vinylacetat produziert, mit der Abänderung, daß ohne Monochloressigsäure gearbeitet wird.
56 000 Nm³/h eines Gasgemisches der folgenden Zusammensetzung:

65 Vol.-%  Ethylen
12 Vol.-%  Essigsäure
10 Vol.-%  $CO_2$
6,5 Vol.-%  Sauerstoff
6,5 Vol.-%  Inerte (Stickstoff, Argon, Ethan) und Wasser

werden über den Kontakt geleitet. Zur Aufrechterhaltung der Druckverhältnisse werden 2937 Nm³/h Ethylen und 2392 Nm³ Sauerstoff zugeführt.
Nach Reaktor werden durch Kondensation 23,5 t/h Rohvinylacetat folgender Zusammensetzung gewonnen:

42,6 Gew.-%  Vinylacetat
46,8 Gew.-%  Essigsäure
10,6 Gew.-%  Wasser.

4

**0 048 946**

Daraus ergeben sich folgende spezifische Verbrauchswerte bzw. Ausbeuten:

Ethylen: 370,0 kg/t Vinylacetat, entsprechend 88,0 Gew.-% Ausbeute
Sauerstoff: 341,8 kg/t Vinylacetat, entsprechend 54,4 Gew.-% Ausbeute
Gebildetes $CO_2$: 100,1 kg/t Vinylacetat.

Entsprechend beträgt der Ethylenverbrennungsanteil 0,69 Gew.-%, bezogen auf eingesetztes Ethylen, bzw. 8,6 Gew.-%, bezogen auf umgesetztes Ethylen.

Die Einsatzdauer des Kontaktes betrug 10 000 Stunden, wobei zur Aufrechterhaltung der erforderlichen Raum-Zeit-Ausbeute die Temperatur im Reaktor von anfänglich 165°C allmählich bis auf 200°C angehoben werden mußte.

## Beispiel 2

Es wird Vinylacetat an dem gemäß Beispiel 1 beschriebenen Katalysator hergestellt, wobei die folgende Versuchsapparatur eingesetzt wird:

Ein vertikal angeordnetes Edelstahlrohr von 25 mm lichter Weite und einer Länge von 2500 mm dient als Reaktor, mit 1000 cm³ Katalysator beschickt ist. Am Reaktoraustritt ist ein Intensivkühler angeschlossen zur Abscheidung der kondensierbaren Reaktionsprodukte. Das Kondensat wird gaschromatographisch auf Vinylacetat untersucht. Weiter ist am Austritt des Kühlers eine Gasbürette zur Sammlung von Abgasproben angebracht, um den $CO_2$-Gehalt des Abgases durch gaschromatographische Analyse zu messen. Am Reaktoreintritt werden 3 l/h Sauerstoff, 35 l/h Ethylen und 13 l/h Stickstoff zugegeben. Der Stickstoff- und Ethylenstrom wird über eine mit Essigsäure beaufschlagte, auf 70°C temperierte Frittenflasche geleitet, um partialdruckmäßig Essigsäure mit dem Gasstrom in das Reaktionssystem eingetragen. Der Essigsäure sind erfindungsgemäße organische Chlorverbindungen zugesetzt.

Die Temperatur im Katalysatorbett beträgt konstant 170°C und wird über eine Mantelbeheizung des Reaktionsrohres gesteuert. Die Reaktion wird beim Druck der umgebenden Atmosphäre durchgeführt.

Ergebnisse:

Tabelle 1

| Zusatz zur Essigsäure | Zusatz in ppm, bezogen auf Essigsäure | $CO_2$-Konzentration im Abgas | Konzentration von Vinylacetat im Kondensat |
|---|---|---|---|
| — | — | 4,5 Vol.-% | 1,5 Gew.-% |
| Chloressigsäure | 1000 | 4,0 Vol.-% | 1,8 Gew.-% |
| Chloracetaldehyd | 1000 | 4,0 Vol.-% | 1,8 Gew.-% |
| Chloressigsäure | 5000 | 3,0 Vol.-% | 2,2 Gew.-% |
| Chloracetaldehyd | 5000 | 3,0 Vol.-% | 2,2 Gew.-% |
| $CCl_4$ | 100 | 4,05 Vol.-% | 1,95 Gew.-% |
| $CHCl_3$ | 200 | 3,8 Vol.-% | 2,1 Gew.-% |

## Beispiel 3

Es wird Vinylacetat in der gemäß Beispiel 2 beschriebenen Apparatur hergestellt an 1000 cm³ eines Trägerkatalysators, der in der folgenden Weise hergestellt wird (vgl. hierzu DE-AS 1 277 249):

1000 cm³ Kieselsäureträgermaterial in Kugelform (Durchmesser 4 mm) werden mit einer wäßrigen Lösung getränkt, die 4 g Palladium (als $PdCl_2$) und 1,5 g Gold (als $H(AuCl_4)$) enthält. Anschließend wird mit 5%iger Hydrazinhydratlösung reduziert, getrocknet und danach mit destilliertem Wasser hydrazinfrei gewaschen. Das Produkt wird noch mit einer 11%igen wäßrigen Kalium-Natrium-Acetatlösung (Molverhältnis Natrium zu Kalium gleich 1 : 1) behandelt und schließlich im Vakuum bei 60° getrocknet.

Ergebnisse:

Tabelle 2

| Zusatz zur Essigsäure | Zusatz in ppm, bezogen auf Essigsäure | $CO_2$-Konzentration im Abgas | Konzentration von Vinylacetat im Kondensat |
|---|---|---|---|
| – | – | 4,2 Vol.-% | 1,6 Gew.-% |
| Dichloressigsäure | 5000 | 3,4 Vol.-% | 2,2 Gew.-% |
| Dichloracetaldehyd | 5000 | 3,4 Vol.-% | 2,2 Gew.-% |
| $CF_2Cl_2$ | 50 | 3,3 Vol.-% | 2,05 Gew.-% |
| $C_2H_4Cl_2(1,2)$ | 100 | 3,6 Vol.-% | 1,95 Gew.-% |

Beispiel 4

Es wird Vinylacetat hergestellt in der gemäß Beispiel 2 beschriebenen Anordnung an einem Träger-katalysator auf Basis von Magnesiumspinell mit 2 Gew.-% Palladium und 2 Gew.-% Natriumacetat als aktive Komponenten (vgl. hierzu DE-AS 1 249 255).

Ergebnisse:

Tabelle 3

| Zusatz zur Essigsäure | Zusatz in ppm, bezogen auf Essigsäure | $CO_2$-Konzentration im Abgas | Konzentration von Vinylacetat im Kondensat |
|---|---|---|---|
| – | – | 4,4 Vol.-% | 1,55 Gew.-% |
| $C_2HCl_5$ | 8000 | 3,8 Vol.-% | 1,95 Gew.-% |
| 1,1,2-Trichlorpropan | 5000 | 3,6 Vol.-% | 2,2 Gew.-% |

Die Ergebnisse der Beispiele 2, 3 und 4 zeigen, verglichen mit den jeweils in der ersten Reihe der Tabelle angegebenen Blindproben, daß der Zusatz der erfindungsgemäßen organischen Chlorverbindungen sowohl die Vinylacetatausbeute steigert, als auch die Verbrennungsrate von Ethylen senkt.

Beispiel 5

Es wird Allylacetat hergestellt in der gemäß Beispiel 2 beschriebenen Versuchsanordnung, wobei anstatt Ethylen Propylen als Olefin eingesetzt wird. Es werden die gemäß den Beispielen 2, 3 und 4 beschriebenen Katalysatoren verwendet.

0 048 946

Ergebnisse:

Tabelle 4

| Katalysator | Zusatz zur Essigsäure | Zusatz in ppm, bezogen auf Essigsäure | $CO_2$-Konzentration im Abgas | Allylacetatkonzentration im Kondensat |
|---|---|---|---|---|
| Gemäß Beispiel 1 | – | – | 4,6 Vol.-% | 1,3 Gew.-% |
| Gemäß Beispiel 2 | Chloressigsäure | 5000 | 3,1 Vol.-% | 1,8 Gew.-% |
| Gemäß Beispiel 3 | Chloracetaldehyd | 5000 | 3,1 Vol.-% | 1,8 Gew.-% |
| Gemäß Beispiel 3 | – | – | 4,3 Vol.-% | 1,5 Gew.-% |
| Gemäß Beispiel 4 | – | – | 4,6 Vol.-% | 1,4 Gew.-% |
| Gemäß Beispiel 4 | $CH_2Cl_2$ | 100 | 3,9 Vol.-% | 2,2 Gew.-% |

Die Ergebnisse gemäß Tabelle 4 zeigen wiederum, daß der Zusatz der erfindungsgemäßen organischen Chlorverbindungen die Ausbeute an ungesättigtem Carbonsäureester erhöht und die Verbrennungsrate des Olefins senkt.

**Patentansprüche**

1. Verfahren zur Herstellung von ungesättigten Carbonsäureestern durch Umsetzung von Olefinen oder Cyclo-Olefinen mit Sauerstoff und Carbonsäure in der Gasphase in Gegenwart von Elemente der achten Nebengruppe des Periodensystems und/oder deren Verbindungen, sowie Alkaliacetat enthaltendem Katalysator bei Temperaturen von 100 bis 250°C und Drücken von 0,5 bis 21 bar absolut, dadurch gekennzeichnet, daß die Umsetzung in Gegenwart von 1 bis 8000 Gew.-ppm, bezogen auf eingesetzte Carbonsäure, an solchen organischen Chlorverbindungen, einzeln oder im Gemisch, vorgenommen wird, die ausgewählt sind aus der Gruppe

a)   der $\alpha$-Chlorcarbonsäuren mit 1 bis 4 Kohlenstoffatomen und deren Ester von Alkoholen mit 1 bis 4 Kohlenstoffatomen,
b)   der $\alpha$-Chloraldehyde mit 1 bis 4 Kohlenstoffatomen,
c)   der Chlorderivate des Methans mit 1 bis 4 Chloratomen,
d)   der Chlorderivate des Methans mit 1 bis 3 Chloratomen, die zumindest ein Fluoratom enthalten, und
e)   der Chlorderivate des Ethans und des Propans, die zumindest 2 Chloratome enthalten.

2. Verfahren nach Anspruch 1, dadurch gekennzeichnet, daß die organischen Chlorverbindungen in Mengen von 10 bis 100 Gew.-ppm, bezogen auf Carbonsäuremenge, eingesetzt werden.
3. Verfahren nach den Ansprüchen 1 und 2, dadurch gekennzeichnet, daß die Zugabe der organischen Chlorverbindungen kontinuierlich erfolgt.
4. Verfahren nach den Ansprüchen 1, 2 und 3, dadurch gekennzeichnet, daß als ungesättigter Carbonsäureester Vinylacetat hergestellt wird.

**Claims**

1. Process for the manufacture of unsaturated carboxylic acid esters by the reaction of olefins or cycloolefins with oxygen and carboxylic acids in the gaseous phase, in the presence of a catalyst which contains elements of subsidiary group VIII of the periodic system and/or compounds thereof, and an alkali metal acetate, at temperatures of from 100 to 250°C and pressures of from 0.5 to 21 bar absolute, characterised in that the reaction is carried out in the presence of from 1 to 8000 parts per million (by weight), calculated on the carboxylic acid used; of organic chlorine compounds, individually or in admixture, selected from the group consisting of

a)   $\alpha$-chlorocarboxylic acids having from 1 to 4 carbon atoms and esters thereof with alcohols having from 1 to 4 carbon atoms,
b)   $\alpha$-chloroaldehydes having from 1 to 4 carbon atoms,

7

c) chlorine derivatives of methane having from 1 to 4 chlorine atoms,
d) chlorine derivatives of methane having from 1 to 3 chlorine atoms and at least one fluorine atom, and
e) chlorine derivatives of ethane and of propane each having at least 2 chlorine atoms.

2. Process according to claim 1, characterised in that the organic chlorine compounds are used in quantities of from 10 to 100 parts per million (by weight), calculated on the quantity of carboxylic acid.

3. Process according to claims 1 and 2, characterised in that the addition of the organic chlorine compounds is carried out continously.

4. Process according to claims 1, 2 and 3, characterised in that vinyl acetate is manufactured as the unsaturated carboxylic acid ester.

**Revendications**

1. Procédé de fabrication d'esters d'acides carboxyliques insaturés par réaction d'oléfines ou de cyclo-oléfines avec de l'oxygène et un acide carboxylique en phase gazeuse, en présence d'un catalyseur comprenant des éléments du sous-groupe 8 de la classification périodique et/ou des composés de ces éléments, ainsi qu'un acétate de métal alcalin, à des températures de 100 à 250°C et sous des pressions abslolues de 0,5 à 21 bars, procédé caractérisé en ce que l'on effectue la réaction en présence de 1 à 8000 ppm en poids, par rapport à l'acide carboxylique employé, de composés organiques chlorés, employés individuellement ou en mélanges, choisis parmi:

a) des acides $\alpha$-chlorocarboxyliques en $C_1$ à $C_4$ et leurs esters avec des alcools en $C_1$ à $C_4$,
b) des $\alpha$-chloro-aldéhydes en $C_1$ à $C_4$,
c) des dérivés chlorés du méthane pouvant avoir de 1 à 4 atomes de chlore,
d) des dérivés chlorés du méthane pouvant avoir de 1 à 3 atomes de chlore, avec au moins un atome de fluor, et
e) des dérivés chlorés de l'éthane et du propane ayant au moins 2 atomes de chlore.

2. Procédé selon la revendication 1, caractérisé en ce que les composés organiques chlorés sont ajoutés à raison de 10 à 100 ppm en poids par rapport à la quantité d'acide carboxylique.

3. Procédé selon la revendication 1 ou 2, caractérisé en ce que l'addition des composés organiques chlorés se fait en continu.

4. Procédé selon l'une quelconque des revendications 1 à 3, caractérisé en ce que l'ester préparé est l'acétate de vinyle.